# FASCICULE DE BREVET EUROPEEN

(11) **EP 3 860 467 B1**
(45) Date de publication et mention de la délivrance du brevet: **30.11.2022**
(21) Numéro de dépôt: 19774121.8
(22) Date de dépôt: 01.10.2019
(51) Int. Cl.: A61B 8/08, A61B 8/00

(54) **PROCEDE DE SELECTION AUTOMATIQUE D'UNE PLAGE DE PROFONDEUR DE CALCUL D'UNE PROPRIETE D'UN MILIEU VISCOELASTIQUE**
VERFAHREN ZUR AUTOMATISCHEN AUSWAHL EINES TIEFENBEREICHS ZUR BERECHNUNG EINER EIGENSCHAFT EINES VISKOELASTISCHEN MEDIUMS
METHOD FOR AUTOMATICALLY SELECTING A DEPTH RANGE FOR CALCULATING A PROPERTY OF A VISCOELASTIC MEDIUM

(30) Priorité: 02.10.2018 FR 1859096
(43) Date de publication de la demande: 11.08.2021
(73) Titulaire: Echosens, 75014 Paris (FR)
(72) Inventeur: SANDRIN, Laurent, 92340 Bourg-la-Reine (FR); CLET, Michel, 75020 Paris (FR)
(74) Mandataire: Cabinet Camus Lebkiri
(86) Numéro de dépôt international: PCT/EP2019/076616
(87) Numéro de publication internationale: WO 2020/070139

(56) Documents cités:
- JP-A- 2016 007 315
- US-A1- 2007 093 716
- VARGHESE T ET AL: "A theoretical framework for performance characterization of elastography: the strain filter", IEEE TRANSACTIONS ON ULTRASONICS, FERROELECTRICS AND FREQUENCY CONTROL, IEEE, US, vol. 44, no. 1, 1 janvier 1997 (1997-01-01), pages 164-172, XP011437533, ISSN: 0885-3010, DOI: 10.1109/58.585212

## Description

### DOMAINE TECHNIQUE

La présente invention appartient au domaine de l'élastographie pour la mesure d'au moins une propriété d'un milieu viscoélastique. Un objet de la présente invention est un procédé de sélection automatique d'une plage de profondeur de calcul lors de la mesure d'une propriété d'un milieu viscoélastique. Le procédé selon l'invention permet d'augmenter la rapidité, fiabilité et reproductibilité de la mesure en sélectionnant la plage de profondeur remplissant un critère de sélection prédéterminé. Un deuxième objet de l'invention est un procédé de mesure globale d'au moins une propriété d'un milieu viscoélastique dans lequel la ou les plages de profondeur de calcul sont sélectionnées automatiquement en utilisant le procédé de sélection automatique selon l'invention. Un troisième objet de l'invention est un dispositif pour la mesure d'une propriété d'un milieu viscoélastique avec sélection automatique de la plage de profondeur de calcul.

### ETAT DE LA TECHNIQUE

L'élastographie transitoire (aussi appelée élastographie impulsionnelle) est une des méthodes les plus connues et efficaces pour déterminer l'élasticité d'un milieu viscoélastique. Par exemple, l'élastographie transitoire est couramment utilisée pour déterminer l'élasticité du foie chez l'homme ou l'animal.

En élastographie transitoire, une onde de cisaillement impulsionnelle est générée et sa vitesse de propagation à l'intérieur du milieu viscoélastique d'intérêt est mesurée. La vitesse de propagation de l'onde de cisaillement permet ensuite de calculer le module de Young du milieu et donc de mesurer son élasticité.

Il existe plusieurs techniques de mise en œuvre de l'élastographie transitoire.

Par exemple, la demanderesse a développé et commercialisé une technique d'élastographie transitoire à vibration contrôlée (ou Vibration Controlled Transient Elastography, « VCTE »). Le dispositif mettant en œuvre cette technique, appelé Fibroscan ^{®}, est capable de mesurer l'élasticité du foie humain de façon rapide, noninvasive et reproductible. Dans un tel dispositif pour élastographie transitoire, l'onde de cisaillement est générée par un vibreur placé au contact du milieu à caractériser. La propagation de l'onde de cisaillement est ensuite suivie à l'aide d'une série d'acquisitions ultrasonores réalisées par un transducteur ultrasonore avec un taux de répétition élevé. Chaque acquisition ultrasonore correspond à au moins une émission ultrasonore. Chaque émission ultrasonore peut être associée à la détection et l'enregistrement à la volée des échos générés par les particules réfléchissantes présentes dans le milieu étudié pour une gamme de profondeurs définie. Les signaux ultrasonores réfléchis sont traités par corrélation pour remonter aux mouvements du tissu engendrés par la propagation de l'onde de cisaillement en fonction du temps et de la position dans le milieu. L'étude de ces mouvements permet de remonter à la vitesse de propagation de l'onde de cisaillement à l'intérieur du milieu viscoélastique, et ainsi à l'élasticité des tissus, comme il est expliqué dans le document « Transient Elastography : a new noninvasive method for assessment of hepatic fibrosis » de L. Sandrin et al., publié dans Ultrasound in Medecine and Biology, Vol. 29, pages 1705-1713, 2003.

La publication US 2007/093716 A1 divulgue un dispositif capable de fournir à l'opérateur une rétroaction en temps réel sur la qualité et la quantité de la compression des tissus.

Dans le cas de la mesure d'une propriété viscoélastique d'un milieu tel qu'un foie humain, il est nécessaire de sélectionner la portion d'ultrasons réfléchis correspondants à la profondeur à laquelle se situe le milieu.

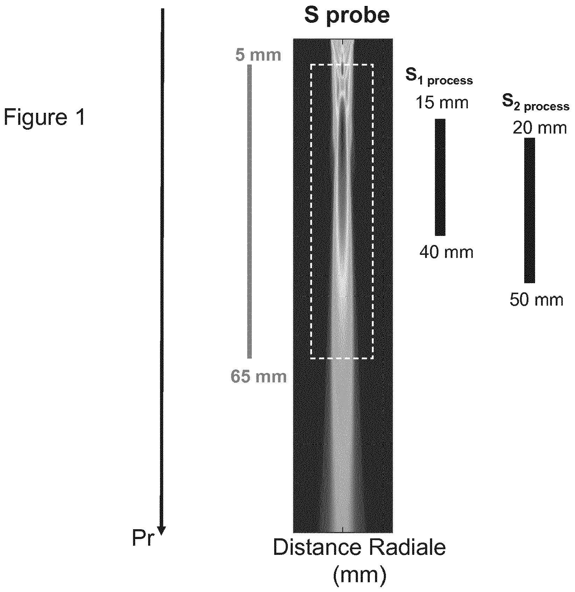

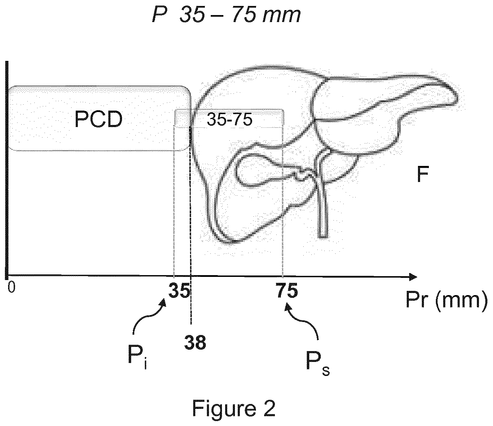

Cette opération de sélection est compliquée par le fait que cette profondeur varie fortement selon la morphologie du patient. Par exemple, dans le cas d'un patient obèse, le milieu viscoélastique à analyser se trouve plus probablement à une profondeur plus grande que dans le cas d'un patient ayant une morphologie normale. Pour cette raison, dans le cas d'une mesure d'élasticité effectuée à l'aide du Fibroscan ^{®}, plusieurs plages de profondeur de calcul sont possibles. La figure 1 représente la distribution de la puissance acoustique des ultrasons à l'intérieur du corps du patient pour une sonde de taille S et en fonction de la profondeur Pr à l'intérieur du corps. Par exemple, il est possible de sélectionner une plage de profondeur de calcul parmi deux possibles plages S1 et S2. S1 correspond à la plage de profondeur comprise entre 15 mm et 40 mm. S2 correspond à la plage de profondeur comprise entre 20 et 50 mm. La figure 2 illustre le cas d'une mesure d'une propriété du foie utilisant une plage de profondeur comprise entre 35 mm et 75 mm. Dans l'exemple de la figure 2, la distance entre la sonde et la paroi extérieure du foie (ou probe-to-capsula distance, PCD, selon la terminologie anglaise) est égale à 38 mm. Si la plage de profondeur à laquelle la propriété est calculée est comprise entre 35 mm et 75 mm, celle-ci n'est pas optimale car elle n'est pas entièrement incluse à l'intérieur du foie. En particulier, la présence de la paroi externe du foie ou capsule du foie à l'intérieur de la plage de calcul peut fausser la mesure.

Dans les solutions actuellement connues de l'homme du métier, la profondeur à laquelle calculer une propriété viscoélastique est fixée ou son choix est laissé à l'opérateur. Cela peut fausser la mesure, si la plage de profondeur fixée n'est pas adaptée à la morphologie du patient, ou la rendre dépendante de l'opérateur en réduisant sa fiabilité et sa reproductibilité.

Par ailleurs, un changement de plage de profondeur de la part de l'opérateur entraîne l'effacement de toutes les mesures. Il en résulte que la durée de l'examen peut augmenter considérablement.

En d'autres termes, les solutions techniques existantes ne permettent pas une sélection optimale et automatique de la plage de profondeur de calcul lors de la mesure d'une propriété d'un milieu viscoélastique tel qu'un foie humain ou animal.

### RESUME GENERAL DE L'INVENTION

Pour résoudre au moins partiellement les problèmes de l'état de la technique, un objet de la présente invention est un procédé de sélection automatique d'une plage de profondeur de calcul d'une propriété d'un milieu viscoélastique, la plage de profondeur étant choisie parmi P plages possibles, P étant un nombre entier supérieur ou égal à 2, ledit procédé comprenant les étapes suivantes :
- Calculer, à partir d'un signal ultrasonore acquis à l'aide d'une sonde pour élastographie, la valeur de la propriété du milieu viscoélastique dans au moins une des P plages de profondeur possibles et la distance entre la sonde et la paroi délimitant le milieu viscoélastique ;
- Déterminer la validité d'au moins une des P plages de profondeur de calcul, une plage de profondeur de calcul étant considérée valide si elle remplit un critère de validité calculé à partir de la distance entre la sonde et la paroi délimitant le milieu viscoélastique ;

- Déterminer la validité du calcul de la propriété du milieu viscoélastique sur la ou les plages de profondeur de calcul valides, ledit calcul de la valeur étant considérée valide s'il remplit un critère de validité calculé à partir de la qualité d'un élastogramme ;
- Sélectionner, parmi les plages de profondeur valides comprenant au moins une valeur de la propriété du milieu viscoélastique dont le calcul est valide, une plage de profondeur remplissant un critère de sélection prédéterminé.

On entend par propriété d'un milieu viscoélastique, une propriété viscoélastique ou ultrasonore du milieu viscoélastique. Un exemple de propriété viscoélastique est la vitesse de propagation d'une onde de cisaillement à l'intérieur du milieu viscoélastique. Un autre exemple de propriété viscoélastique du milieu est l'élasticité du milieu viscoélastique. Un exemple de propriété ultrasonore du milieu viscoélastique est un paramètre d'atténuation ultrasonore à l'intérieur du milieu viscoélastique.

On entend par sonde pour élastographie, une sonde comprenant au moins un transducteur ultrasonore. Ladite sonde peut être utilisée pour réaliser des mesures des propriétés viscoélastiques d'un milieu. Un exemple de sonde pour élastographie est une sonde configurée pour réaliser une mesure d'élastographie transitoire.

On entend par mesure d'une propriété viscoélastique l'ensemble des étapes du procédé permettant de fournir une valeur de la propriété du milieu viscoélastique, de l'étape de calcul de la propriété à l'étape de sélection automatique de la plage de profondeur optimale.

On entend par mesure globale d'une propriété du milieu viscoélastique la répétition du procédé de mesure précédemment défini sur M mesures et le calcul global de la valeur de la propriété. Le calcul global peut être réalisé à l'aide d'une fonction mathématique de type médiane ou moyenne sur les valeurs de la propriété dont le calcul est valide. On entend par profondeur une direction de l'espace s'étendant à l'intérieur du milieu viscoélastique d'intérêt. Par exemple, la profondeur est la direction correspondant à la direction de propagation des ultrasons émis pendant la mesure de la propriété du milieu viscoélastique. Quand le milieu est un foie humain, la peau du patient correspond à la profondeur zéro. La sonde pour élastographie étant au contact de la peau du patient, la distance peau-capsule du foie correspond à la distance sonde-capsule du foie.

On entend par plage de profondeur de calcul l'intervalle de profondeur à l'intérieur duquel la propriété est calculée. Par exemple, si la propriété viscoélastique est mesurée au moyen d'une ou plusieurs acquisitions ultrasonores, la plage de profondeur de calcul est la région à l'intérieur de laquelle sont réfléchis les signaux ultrasonores détectés et utilisés pour le calcul de la propriété.

L'étape de calcul de la valeur d'une propriété du milieu viscoélastique peut comprendre une étape de mesure par élastographie transitoire comportant le calcul d'un élastogramme.

On entend par élastogramme l'évolution d'un paramètre de déplacement en fonction du temps et de la profondeur représentant la propagation de l'onde de cisaillement dans le milieu viscoélastique.

L'étape de calcul peut également comprendre une série d'acquisitions ultrasonores comportant le calcul de la distance entre la sonde et la paroi délimitant le milieu viscoélastique, chaque acquisition comprenant l'émission d'une impulsion ultrasonore et la détection à la volée des signaux ultrasonores réfléchis. Lors de l'étape de calcul, la valeur de la propriété viscoélastique peut être calculée dans une plage de profondeur ou dans plusieurs plages de profondeurs possibles.

On entend par critère de validité de la plage de profondeur, un critère calculé à partir de la distance entre la sonde ultrasonore et la paroi du milieu viscoélastique. Cette distance est également appelée « probe-to-capsula distance » ou PCD selon la terminologie anglaise.

Une plage de profondeur est définie valide si elle ne comprend pas la paroi ou surface du milieu viscoélastique. Par exemple, si le milieu viscoélastique à caractériser est un foie humain ou animal, une plage de profondeur est définie valide si elle ne comprend pas la capsule du foie. En d'autres termes, une plage de profondeur est définie valide si elle est entièrement comprise dans le milieu à caractériser.

On entend par critère de validité du calcul de la propriété du milieu viscoélastique un critère établi à partir d'un élastogramme construit à partir d'une acquisition ultrasonore. Selon un mode de réalisation, le critère de validité du calcul de la propriété du milieu viscoélastique sur une plage de profondeur donnée peut prendre en compte la qualité de la propagation de l'onde de cisaillement. Il peut correspondre par exemple au coefficient de détermination de la régression linéaire obtenue à partir de la propagation de l'onde de cisaillement représentée dans l'élastogramme (voir article Ultrasound in Medicine and Biology, vol 29, numéro 12, 2003, page 3) ou au rapport signal à bruit de l'élastogramme. Dans ces cas, le calcul de la propriété est considéré valide dans une plage de profondeur donnée uniquement si le coefficient de détermination ou le rapport signal à bruit est supérieur à une valeur prédéterminée.

Si plusieurs plages de profondeurs sont valides, un critère de sélection est appliqué sur les valeurs de la propriété dont le calcul est valide pour choisir automatiquement la plage de profondeur optimale.

Avantageusement, le procédé selon l'invention permet d'écarter les plages de profondeur qui ne sont pas entièrement comprises à l'intérieur du milieu à caractériser. En d'autres termes, le calcul de la propriété est automatiquement réalisé à l'intérieur de l'organe ou milieu à caractériser.

Avantageusement, le procédé selon l'invention permet d'écarter les valeurs de la propriété pour lesquelles la propagation de l'onde de cisaillement est de mauvaise qualité sur l'élastogramme. Le calcul de la vitesse de propagation de cette onde de cisaillement est ainsi plus fiable.

Avantageusement, le procédé de sélection automatique de la plage de profondeur de calcul rend la mesure morpho-adaptive. En effet, la plage de profondeur retenue par le procédé selon l'invention se trouve automatiquement à l'intérieur de l'organe, indépendamment de la morphologie du patient.

Avantageusement, le procédé de sélection automatique de la plage de profondeur permet de s'affranchir au moins en partie de la dépendance de l'opérateur. En d'autres termes, la sélection de la plage de profondeur de calcul n'est plus effectuée par l'opérateur.

Avantageusement, grâce au procédé selon l'invention, la mesure couvre une zone plus grande du milieu viscoélastique à caractériser.

Avantageusement, quand le milieu viscoélastique est un organe, par exemple un foie humain ou animal, l'examen est plus rapide notamment pour les patients présentant une distance sonde-paroi de l'organe ou PCD importante.

Si plusieurs plages de profondeurs sont valides, un critère de sélection est appliqué lors de l'étape de sélection afin de choisir une plage de profondeur de calcul. Il existe plusieurs critères de sélection de la plage de profondeur.

Le procédé selon l'invention comprend en outre une étape de détermination de la validité du calcul de la propriété du milieu viscoélastique, ce calcul étant considérée valide s'il remplit un critère de validité calculé à partir de la qualité d'un élastogramme.

Les critères de sélection de la plage de profondeur ne sont appliqués que sur les valeurs de la propriété dont le calcul est valide sur au moins une plage de profondeurs valide.

Le procédé est mise en œuvre à chaque nouvelle mesure M. Lors de la mise en œuvre du procédé, le nombre de calculs de la propriété du milieu viscoélastique est au moins égal à 1, et au plus égale à P.

Lors de l'étape de détermination de la validité de la plage de profondeur de calcul, la validité de chacune des P plages de profondeurs est déterminée à chaque nouvelle mesure M.

Lors de l'étape de détermination de la validité du calcul de la propriété du milieu viscoélastique, la validité de chacun des calculs de ladite propriété est déterminée à chaque nouvelle mesure M.

Selon un mode de réalisation, le critère de sélection repose uniquement sur la dernière mesure. En d'autres termes, le procédé selon l'invention ne prend en compte que l'information disponible au moment de la dernière mesure pour sélectionner la plage de profondeur.

Dans ce cas, la plage de profondeur sélectionnée lors de l'étape de sélection peut être la plage de profondeur pour laquelle on observe le plus grand rapport signal sur bruit dans l'élastogramme calculé.

Selon un mode de réalisation, la plage de profondeur sélectionnée lors de l'étape de sélection est la plage de profondeur dans laquelle l'élastogramme est de meilleure qualité.

Selon un mode de réalisation la plage de profondeur sélectionnée lors de l'étape de sélection est celle qui remplit un critère calculé à partir de l'homogénéité du milieu. L'homogénéité du milieu peut être mesurée à partir de la série d'acquisition du signal ultrasonore.

Avantageusement, ces critères permettent de sélectionner une plage de profondeur dans laquelle l'onde de cisaillement se propage correctement.

Selon un mode de réalisation, si le calcul de la propriété du milieu viscoélastique n'est pas valide dans une plage de profondeur, la plage de profondeur suivante est sélectionnée jusqu'à obtenir un calcul de la propriété valide. Si aucun calcul de la propriété n'est caractérisé valide parmi toutes les plages de profondeurs valides, une nouvelle mesure est réalisée, sinon, au moins un des critères de sélection précédent doit être rempli. Cela permet d'observer un segment plus large de l'organe et d'optimiser le temps d'examen.

Selon un mode de réalisation, le critère de sélection repose sur l'ensemble des mesures réalisées. En d'autres termes, le procédé selon l'invention prend en compte l'information disponible de la première à la dernière mesure pour sélectionner la plage de profondeur.

Selon un mode de réalisation, la plage de profondeur sélectionnée lors de l'étape de sélection est celle pour laquelle on obtient le plus grand nombre de calculs de la propriété valides parmi les M mesures effectuées.

Selon un mode de réalisation, la plage de profondeur sélectionnée lors de l'étape de sélection est celle pour laquelle on obtient une minimisation de la dispersion des M valeurs calculées dans chaque plage. Par exemple, la dispersion des valeurs calculées peut être donnée par l'écart interquartile ou par l'écart-type des valeurs calculées.

Selon un mode de réalisation, la plage de profondeur sélectionnée lors de l'étape de sélection est celle pour laquelle on obtient le meilleur rapport signal à bruit sur les M élastogrammes calculés.

Selon un mode de réalisation, la plage de profondeur sélectionnée lors de l'étape de sélection est celle dans laquelle les M élastogrammes calculés comportent la meilleure qualité.

Avantageusement, la qualité de l'élastogramme jauge la bonne propagation de l'onde de cisaillement dans une plage de profondeur du milieu viscoélastique.

Selon un mode de réalisation, la plage de profondeur sélectionnée lors de l'étape de sélection est celle qui remplit un critère calculé à partir de l'homogénéité du milieu.

L'homogénéité du milieu peut être mesurée à partir de la série d'acquisition du signal ultrasonore.

Le procédé de sélection automatique de la plage de profondeur lors de la mesure d'une propriété d'un milieu viscoélastique selon l'invention peut également présenter une ou plusieurs des caractéristiques ci-dessous, considérées individuellement ou selon toutes les combinaisons techniquement possibles :
- chaque plage de profondeur est délimitée par une première profondeur et une deuxième profondeur et la plage de profondeur est définie valide si la distance entre la sonde et la paroi délimitant le milieu viscoélastique est inférieure à la première et à la deuxième profondeur ;
- un calcul de la propriété du milieu viscoélastique est défini valide dans la plage de profondeur considérée s'il remplit un critère de validité calculé à partir de la qualité d'un élastogramme ;
- lors de l'étape de calcul, la propriété du milieu viscoélastique est calculée dans au moins une des P plages de profondeur possibles ;
- lors de l'étape de calcul, la propriété du milieu viscoélastique est calculée à partir de M mesures réalisées dans au moins une des P plages de profondeur possibles, M étant un nombre entier supérieur ou égal à 2 ;
- le critère de sélection de la plage de profondeur de calcul repose uniquement sur la dernière mesure réalisée ;
- la plage de profondeur sélectionnée lors de l'étape de sélection est celle dans laquelle l'élastogramme calculé comporte le plus grand rapport signal à bruit ;
- la plage de profondeur sélectionnée lors de l'étape de sélection est celle pour laquelle l'élastogramme est de meilleure qualité ;
- la plage de profondeur sélectionnée lors de l'étape de sélection est celle qui remplit un critère déterminé à partir de l'homogénéité du milieu ;
- le critère de sélection repose sur l'ensemble des mesures réalisées ;
- la plage de profondeur sélectionnée lors de l'étape de sélection est celle qui minimise la dispersion entre les valeurs calculées de la propriété lors de l'étape de calcul ;
- la plage de profondeur sélectionnée est celle qui maximise le nombre de calculs valides de la propriété ;
- la plage de profondeur sélectionnée lors de l'étape de sélection est celle dans laquelle les élastogrammes calculées comportent le plus grand rapport signal à bruit ;
- la plage de profondeur sélectionnée lors de l'étape de sélection est celle pour laquelle les élastogrammes sont de meilleure qualité ;
- la plage de profondeur sélectionnée est celle qui remplit un critère de qualité déterminé à partir de l'homogénéité du milieu ;
- si la plage de profondeur dans laquelle la valeur de la propriété viscoélastique a été calculée n'est pas valide, le calcul de de la propriété est réalisée à une plage plus profonde ;
- si, lors de l'étape de sélection, au moins deux plages de profondeurs remplissent le critère de sélection, la plage la plus superficielle est sélectionnée ;
- si, lors de l'étape de sélection, au moins deux plages de profondeurs remplissent le critère de sélection, la plage la plus profonde est sélectionnée ;
- le critère de validité de la plage de profondeur est binaire et peut prendre une valeur correspondant à une plage de profondeur valide ou une valeur correspondante à une plage de profondeur invalide ;
- le critère de validité du calcul de la propriété est binaire et peut prendre une valeur correspondante à un calcul valide ou une valeur correspondante à un calcul invalide ;
- la propriété du milieu viscoélastique est choisie dans un groupe comprenant : l'élasticité du milieu viscoélastique, le module d'Young du milieu viscoélastique, la vitesse de propagation d'une onde de cisaillement à l'intérieur du milieu viscoélastique, le module de cisaillement, un paramètre d'atténuation ultrasonore ou une combinaison de ces propriétés ;
- le critère de validité de la plage de profondeur est un indicateur binaire étant égal à 1 si la plage de profondeur est valide et égal à 0 si la plage de profondeur n'est pas valide ;
- le critère de validité du calcul de la propriété est un indicateur binaire étant égal à 1 si le calcul est valide et égal à 0 si le calcul n'est pas valide.

Un deuxième objet de la présente invention est un procédé de mesure globale d'au moins une propriété d'un milieu viscoélastique. Le procédé de mesure globale comporte une première étape de sélection automatique de la plage de profondeur de calcul en utilisant le procédé de sélection automatique selon l'invention. Les valeurs de la propriété du milieu viscoélastique calculées lors de l'étape de sélection de la plage de profondeur sont stockées, par exemple dans une mémoire.

Le procédé de mesure globale comporte en outre une deuxième étape de calcul global de la valeur de la propriété du milieu viscoélastique à partir des valeurs de la propriété dont le calcul est valide aux plages de profondeur sélectionnées, le calcul étant réalisé à l'aide d'une fonction mathématique de type médiane ou moyenne.

Le calcul global peut être réalisé à l'aide d'une fonction mathématique de type médiane ou moyenne. Selon un mode de réalisation, la première étape de sélection automatique de la plage de profondeur est répétée à chaque nouvelle mesure, aux plages de profondeurs valides et sur les valeurs de la propriété dont le calcul est valide.

Dans le cas où la sélection de la plage de profondeur repose uniquement sur la dernière mesure, la valeur globale de la propriété du milieu viscoélastique est calculée en tant que médiane ou moyenne de l'ensemble des valeurs de la propriété dont les calculs sont valides, sur les plages de profondeurs précédemment sélectionnées et potentiellement différentes. A chaque nouvelle mesure, la plage de profondeur sélectionnée est indépendante des précédentes.

Dans le cas où la sélection de la plage de profondeur repose sur l'ensemble des mesures réalisées, la valeur globale de la propriété du milieu viscoélastique est calculée en tant que médiane ou moyenne de l'ensemble des valeurs de la propriété dont les calculs sont valides, sur la même plage de profondeur. La propriété du milieu viscoélastique est choisie dans un groupe comprenant : l'élasticité, le module d'Young, le module de cisaillement, la vitesse de cisaillement à l'intérieur du milieu viscoélastique, un paramètre d'atténuation ultrasonore ou une combinaison de ces propriétés

Un troisième objet de la présente invention est un dispositif pour la mesure d'une propriété viscoélastique d'un milieu viscoélastique avec sélection automatique de la plage de profondeur de calcul, ledit dispositif comprenant :
- une sonde pour élastographie ;
- des moyens de calcul comprenant au moins une mémoire et un microprocesseur ;
ledit dispositif étant construit et agencé pour :
- Calculer, à partir du signal ultrasonore acquis à l'aide de la sonde pour élastographie et des moyens de calcul, la distance entre la sonde et la paroi délimitant le milieu viscoélastique ;
- Calculer, à partir du signal ultrasonore acquis avec à l'aide de la sonde pour élastographie et des moyens de calcul, la propriété du milieu viscoélastique dans au moins une des P plages de profondeur de calcul possibles ;
- Déterminer la validité d'au moins une des P plages de profondeur de calcul, une plage de profondeur de calcul étant considérée valide si elle remplit un critère de validité calculé à partir de la distance entre la sonde et la paroi délimitant le milieu viscoélastique ;
- Déterminer la validité du calcul de la valeur de la propriété du milieu viscoélastique sur la ou les plages de profondeur valides, ledit calcul étant considérée valide s'il remplit un critère de validité déterminé à partir de la qualité d'un élastogramme ;
- Sélectionner, parmi les plages de profondeurs valides comprenant au moins une valeur de la propriété dont le calcul est valide, une plage de profondeur remplissant un critère de sélection prédéterminé ;
- Calculer, à partir des valeurs de la propriété dont le calcul est valide aux plages de profondeur sélectionnées, la valeur globale de la propriété viscoélastique, la valeur globale étant calculée à l'aide d'une fonction mathématique de type médiane ou moyenne.

Avantageusement le dispositif selon l'invention permet de mesurer une propriété viscoélastique d'un milieu viscoélastique en sélectionnant automatiquement la plage de profondeur de calcul optimale. La sélection automatique de la plage de profondeur est réalisée à l'aide du procédé de sélection de la plage de profondeur selon l'invention. Cela rend la mesure de la propriété viscoélastique fiable, reproductible et indépendante de l'opérateur.

Le dispositif selon l'invention peut également présenter une ou plusieurs des caractéristiques ci-dessous, considérées individuellement ou selon toutes les combinaisons techniquement possibles :
- la sonde pour élastographie est une sonde pour élastographie transitoire ;
- le dispositif selon l'invention et les moyens de calcul sont compris dans la sonde pour élastographie ;
- le dispositif selon l'invention comprend en outre des moyens d'affichage des résultats des mesures. Par exemple les moyens d'affichage sont configurés pour afficher l'élastogramme mesuré, la plage de profondeur retenue pour la mesure et la valeur de la propriété viscoélastique mesurée.

### LISTE DES FIGURES

D'autres caractéristiques et avantages de l'invention ressortiront clairement de la description qui en est donnée ci-dessous, à titre indicatif et nullement limitatif, en référence aux figures parmi lesquelles :
- La figure 1 illustre la distribution de la puissance acoustique des ondes ultrasonores propagées à l'intérieur d'un milieu viscoélastique d'intérêt pendant une mesure de type élastographie impulsionnelle, l'opérateur pouvant choisir parmi deux plages de profondeur S1 et S2 ;
- La figure 2 illustre la mesure d'une propriété d'un milieu viscoélastique tel qu'un foie selon l'état de l'art : la plage de profondeur de calcul peut comprendre la paroi du milieu ;
- La figure 3 illustre schématiquement les étapes du procédé selon l'invention ;
- La figure 4 illustre un choix optimal de la plage de profondeur de calcul lors de la mesure d'une propriété d'un milieu viscoélastique selon l'invention ;
- Les figures 5a à 5d illustrent des exemples de validation des plages de profondeur pour différentes distances sonde-capsule PCD ;
- Les figures 6a et 6b illustrent un exemple de validation d'un calcul de valeur de la propriété du milieu viscoélastique sur P=3 plages de profondeurs ;
- Les figures 7a et 7b illustrent un exemple de validation d'un calcul de valeur de la propriété et de sélection automatique de la plage de profondeur de calcul, le procédé selon l'invention étant mis en œuvre avec P=3 plages de profondeur possibles, le critère de validité pouvant reposer sur la dernière mesure uniquement ou sur l'ensemble des mesures réalisées ;
- La figure 8 illustre schématiquement les étapes du procédé de mesure globale d'une propriété d'un milieu viscoélastique avec sélection automatique de la plage de profondeur de calcul et du calcul global de la valeur de la propriété ;
- La figure 9 résume le procédé PRO de sélection automatique de la plage de profondeur de calcul selon les différents critères de sélection.

### DESCRIPTION DETAILLEE

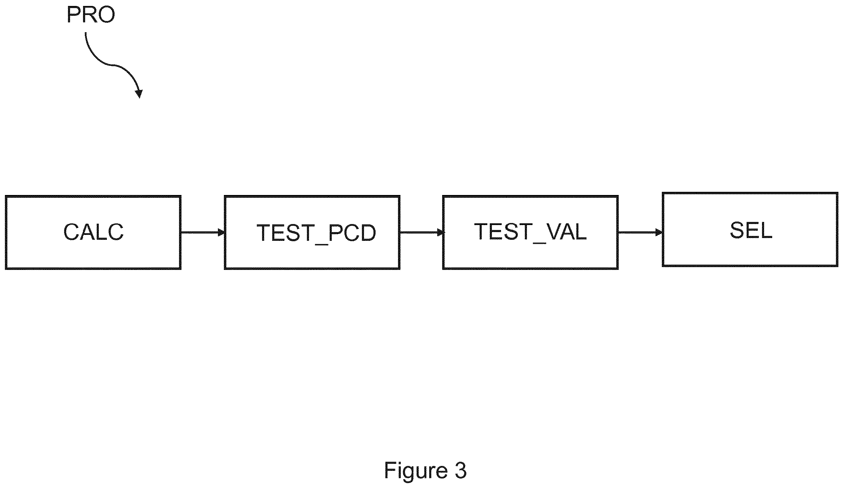

La figure 3 illustre schématiquement les étapes du procédé PRO selon l'invention.

Le procédé PRO selon l'invention comprend les étapes suivantes :
- Une étape CALC de calcul, à partir du signal ultrasonore acquis à l'aide d'une sonde pour élastographie, d'une propriété viscoélastique dans au moins une des P plages de profondeur de calculs possibles. Lors de cette étape la propriété peut être calculée dans une seule plage de profondeur ou dans plusieurs plages de profondeurs ; lors de cette étape, le signal ultrasonore acquis à l'aide d'une sonde pour élastographie est utilisé pour calculer la distance PCD entre la sonde pour élastographie et la paroi du milieu viscoélastique ;
- Une étape TEST_PCD de validation des plages de profondeurs de calcul dans lesquelles la propriété viscoélastique a été calculée. Une plage de profondeur est définie valide si elle remplit un critère calculé à partir de la distance entre la sonde et la paroi du milieu ou « probe to capsula distance », PCD, selon la terminologie anglaise ;
- Une étape TEST_VAL de validation des calculs de la valeur de la propriété du milieu viscoélastique sur la ou les plages de profondeur de calcul valides. Lors de cette étape, un critère de validation est appliqué à chaque calcul de la propriété viscoélastique pour en déterminer la validité. Un calcul est considéré valide s'il remplit un critère de validité calculé à partir de la qualité d'un élastogramme. Par exemple un calcul est considéré valide si la qualité de l'élastogramme mesuré dans la plage de profondeur de calcul est suffisamment élevée. En d'autres termes, un calcul est considéré valide si le rapport signal à bruit de l'élastogramme mesurée est suffisamment élevé ;
- Une étape SEL de sélection d'une plage de profondeur parmi les plages de profondeur valides comprenant au moins un calcul valide selon un critère de sélection. Le critère de sélection peut reposer uniquement sur la dernière mesure réalisée ou sur l'ensemble des mesures effectuées. En d'autres termes, si l'étape de calcul CALC comporte la réalisation d'une seule mesure, le critère de sélection repose uniquement sur la dernière mesure effectuée ou mesure courante. Si l'étape de calcul CALC comporte la réalisation d'une multiplicité de mesures, le critère de sélection peut prendre en compte les différentes mesures effectuées, à savoir l'historique des mesures effectuées.

Ces étapes peuvent être réalisées en suivant l'ordre de la figure 3 ou en suivant un ordre différent. Les étapes, toutes ou en partie, peuvent être réalisées en parallèle.

Selon un mode de réalisation, le critère de sélection de la plage de profondeur de calcul repose uniquement sur la mesure courante. Dans ce cas, la sélection de la plage de profondeur optimale ne prend en compte que l'information fournie par la dernière mesure réalisée.

La plage de profondeur sélectionnée lors de l'étape de sélection SEL peut être :
- celle pour laquelle on observe le meilleur rapport signal à bruit dans l'élastogramme mesuré ;
- celle pour laquelle on observe la meilleure propagation de l'onde de cisaillement sur l'élastogramme, à savoir l'élastogramme de meilleure qualité ;
- celle pour laquelle on observe la plus grande homogénéité du milieu.

Avantageusement, ces critères de sélection de la plage de profondeur permettent de sélectionner, parmi les plages de profondeur à l'intérieur du milieu, la plage de profondeur dans laquelle l'onde de cisaillement se propage correctement ou celle correspondant à une plus grande homogénéité de l'organe.

Selon un autre mode de réalisation, le critère de sélection de la plage de profondeur repose sur l'historique des mesures effectuées. Dans ce cas, la sélection de la plage de profondeur optimale prend en compte l'information fournie par l'ensemble des mesures réalisées.

La plage de profondeur sélectionnée peut être :
- celle pour laquelle on observe le plus grand nombre de mesure valides parmi les M mesures réalisées ;
- celle pour laquelle on observe la plus petite dispersion entre les valeurs de la propriété calculées parmi les M mesures réalisées ;
- celle qui remplit un critère calculé à partir de l'homogénéité du milieu ;
- celle pour laquelle on observe le meilleur rapport signal à bruit moyen ou médian dans les élastogrammes parmi les M mesures réalisées ;
- celle pour laquelle on observe la meilleure propagation de l'onde de cisaillement sur l'élastogramme, c'est-à-dire le meilleur critère de qualité moyen ou médian parmi les M mesures réalisées ;
- celle pour laquelle on observe la plus grande homogénéité du milieu moyenne ou médiane parmi les M mesures réalisées.

La première étape CALC comporte le calcul d'une propriété du milieu dans au moins une des P plages de profondeur possibles, le calcul étant réalisé à partir du signal ultrasonore acquis par une sonde ultrasonore lors d'une mesure. Selon un mode de réalisation, le calcul est répété M fois dans au moins une des P plages.

La propriété mesurée peut être une propriété viscoélastique telle que la vitesse de propagation d'une onde de cisaillement impulsionnelle ou l'élasticité du milieu. Dans ce cas la mesure est une mesure d'élastographie transitoire.

La propriété mesurée peut être une propriété ultrasonore telle qu'un coefficient d'atténuation contrôlé (CAP pour *Coefficient Attenuation Parameter*)*.* Dans ce cas la mesure comporte la génération d'une série d'acquisitions ultrasonores.

Lors de l'étape TEST_PCD, la validité de chaque plage de profondeur de calcul est déterminée. Chaque plage de profondeur de calcul est considérée valide uniquement si le critère de validité remplit une condition prédéterminée.

Le critère de validité de plage de profondeur peut être binaire et possède une première valeur correspondant à une plage valide et une deuxième valeur correspondant à une plage non-valide.

Selon un mode de réalisation, le critère de validité de la plage de profondeur est déterminé à partir de signal ultrasonore réfléchi, sur lequel est calculé la distance entre la sonde ultrasonore et la paroi du milieu viscoélastique. Cette distance est également appelée « probe-to-capsula distance » ou PCD selon la terminologie anglaise.

Une plage de profondeur est définie valide si elle ne comprend pas la paroi ou surface du milieu viscoélastique. Par exemple, si le milieu viscoélastique à caractériser est un foie humain ou animal, une plage de profondeur est définie valide si elle ne comprend pas la capsule du foie.

Avantageusement, un tel critère de validité permet de ne conserver que les mesures entièrement comprises dans le milieu à caractériser.

Lors de l'étape TEST_VAL, la validité du calcul de la valeur de la propriété du milieu viscoélastique sur la ou les plages de profondeur de calcul valides est déterminée. En d'autres termes, la validité de chacune des valeurs calculées est déterminée à chaque nouvelle mesure M. Chaque calcul est considéré valide uniquement si le critère de validité remplit une condition prédéterminée. Le critère de validité du calcul peut être binaire et possède une première valeur correspondant à une mesure valide et une deuxième valeur correspondant à une mesure non-valide.

Selon un mode de réalisation le critère de validité de mesure est défini à partir d'un élastogramme impulsionnel.

L'élastogramme est l'image permettant de visualiser la propagation de l'onde de cisaillement lors d'une mesure d'élastographie impulsionnelle. L'élastogramme est défini par une matrice de deux dimensions et fournit une représentation spatio-temporelle des déplacements engendrés par la propagation de l'onde de cisaillement dans le milieu.

Par exemple, le critère de validité peut être établi à partir de la qualité de l'élastogramme impulsionnel mesuré. Une estimation de la qualité de l'élastogramme impulsionnel peut être fournie par le rapport signal/bruit de l'élastogramme impulsionnel. Les valeurs de la propriété dont le calcul est valide sont alors les mesures avec un élastogramme impulsionnel possédant un rapport signal/bruit supérieur à un seuil prédéterminé.

Un calcul de la propriété du milieu est automatiquement considéré invalide sur une plage de profondeur invalide.

Avantageusement, un tel critère de validité permet d'écarter les mesures correspondant à un élastogramme impulsionnel de mauvaise qualité, par exemple à cause d'un mauvais positionnement de la sonde ou d'une mauvaise propagation de l'onde de cisaillement transitoire à l'intérieur du milieu viscoélastique.

Lors de l'étape SEL, la plage de profondeur optimale est sélectionnée parmi la ou les valeurs de la propriété dont le calcul est valide. Une plage de profondeur est définie optimale si elle remplit le meilleur critère de sélection imposé.

Avantageusement, cette étape permet la sélection automatique de la plage de profondeur optimale pour mesurer la propriété du milieu.

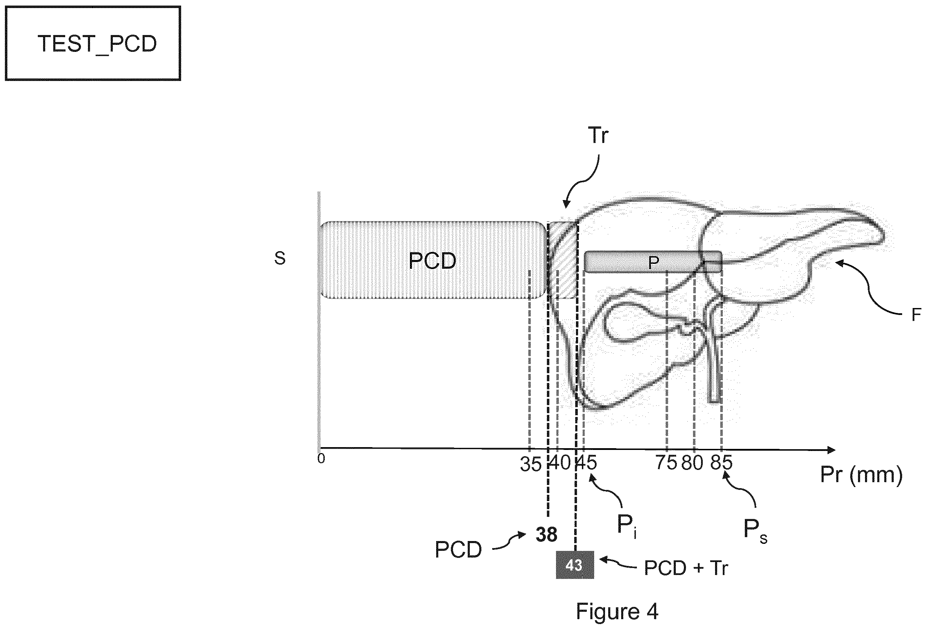

La figure 4 illustre la sélection de la plage de profondeur de calcul selon l'invention quand le milieu viscoélastique est un foie F et le critère de validité d'une plage de profondeur est déterminé à partir d'une mesure ultrasonore, à savoir la distance sonde-capsule PCD séparant la peau S du patient et la paroi du foie. L'axe horizontal de la figure représente la profondeur Pr mesurée à partir de la peau S du patient.

Dans le cas illustré à la figure 4 la distance sonde-capsule PCD mesurée grâce à une ou plusieurs acquisitions ultrasonores est égale à 38 mm.

La plage de profondeur de calcul P possède une borne inférieure Pi et une borne supérieur Ps. Dans le cas de la figure 4, Pi = 45 mm et Ps = 85 mm.

Afin d'obtenir une plage de profondeur valide, la plage de profondeur de calcul P est choisie de façon que sa borne inférieure Pi soit strictement supérieure à la distance sonde-capsule PCD, Pi > PCD.

En d'autres termes, si Pi > PCD, le critère de validité prend la valeur correspondante à une plage de profondeur valide. En cas contraire, le critère de validité prend la valeur correspondante à une plage de profondeur non-valide.

Pour augmenter la fiabilité du critère de validité d'une plage de profondeur il est possible d'introduire une zone de transition Tr. La condition de validité d'une plage de profondeur dans la plage de profondeur P devient alors : Pi > PCD + Tr.

Dans l'exemple de la figure 4 la zone de transition a une épaisseur de 5 mm, Tr = 5 mm.

Avantageusement, l'utilisation d'un critère de validité d'une plage de profondeur défini à partir de la distance sonde capsule PCD permet de s'assurer que la plage de profondeur de calcul est entièrement comprise à l'intérieur du milieu à caractériser.

En d'autres termes, l'utilisation d'un critère de validité d'une plage de profondeur défini à partir de la distance sonde-capsule PCD permet d'éviter des erreurs de mesure liées à la présence de la paroi du milieu viscoélastique à l'intérieur de la zone de mesure.

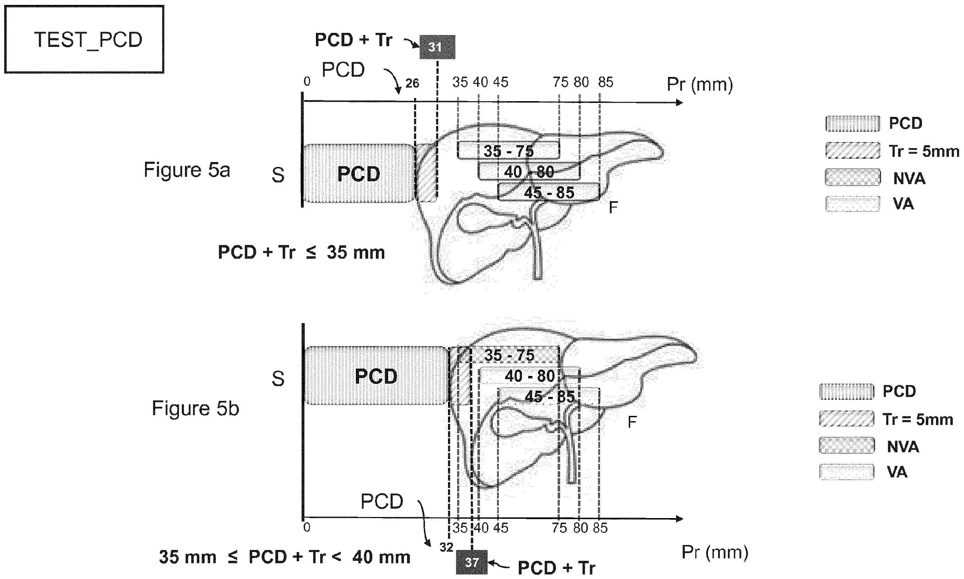

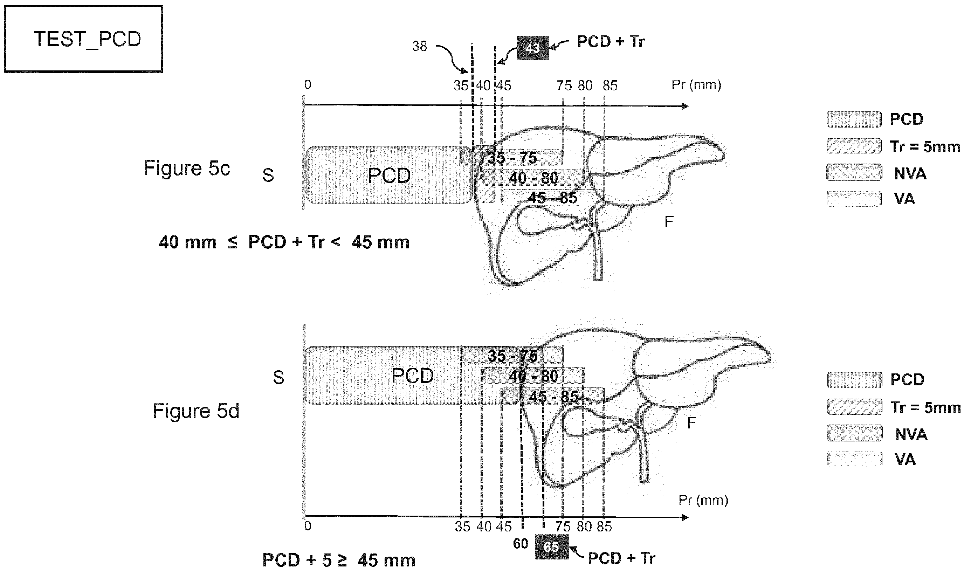

Les figures 5a, 5b, 5c et 5d illustrent la validation des plages de profondeurs de mesure d'une propriété élastique d'un foie F quand trois plages de profondeur sont possibles. Les trois plages de profondeurs représentées sont :
- P[35-75] comprise entre 35 mm et 75 mm de profondeur ;
- P[40-80] comprise entre 40 mm et 80 mm de profondeur ;
- P[45-85] comprise entre 45 mm et 85 mm de profondeur.

La figure 5a correspond à une distance sonde-capsule PCD = 26 mm. En utilisant le critère de qualité d'une plage de profondeur illustré en référence à la figure 4, on obtient que 35 mm > PCD + Tr, 35 mm étant la borne inférieure de la plage la plus superficielle. Dans ce cas les trois plages de profondeurs correspondent à une plage de profondeur valide VA.

La figure 5b correspond à une distance sonde-capsule PCD =32 mm. En utilisant le critère de qualité illustré en référence à la figure 4, on obtient que 35 mm < PCD + Tr < 40 mm. La plage de profondeur P[35-75] possède une borne inférieure égale à 35 mm et correspond donc à une plage de profondeur non valide NVA. Cela est dû au fait que la profondeur correspondante à PCD + TR = 37 mm tombe à l'intérieur de la plage P[35-75]. En d'autres termes, la mesure d'une propriété du foie effectuée en correspondance de la plage P[35-75] serait modifiée par la présence de la paroi du foie et de la zone de transition Tr. En revanche, les plages de profondeur P[40-80] et P[45-85] correspondent à des plages de profondeur valides car elles sont entièrement comprises dans le milieu à caractériser F.

La figure 5c illustre le cas d'une distance sonde-capsule PCD =38 mm. La profondeur PCD + Tr = 43 est plus grande que les bornes inférieures des plages P[35-75] et P[40-80]. Ces deux plages de profondeur correspondent donc à des plages de profondeur non valides NVA. Dans le cas illustré à la figure 5c seule la plage P[45-85] correspond à une plage de profondeur valide VA.

La figure 5d illustre le cas d'une distance sonde-capsule PCD = 60 mm. Dans ce cas, aucune des plages de profondeur ne correspond à une plage de profondeur valide VA.

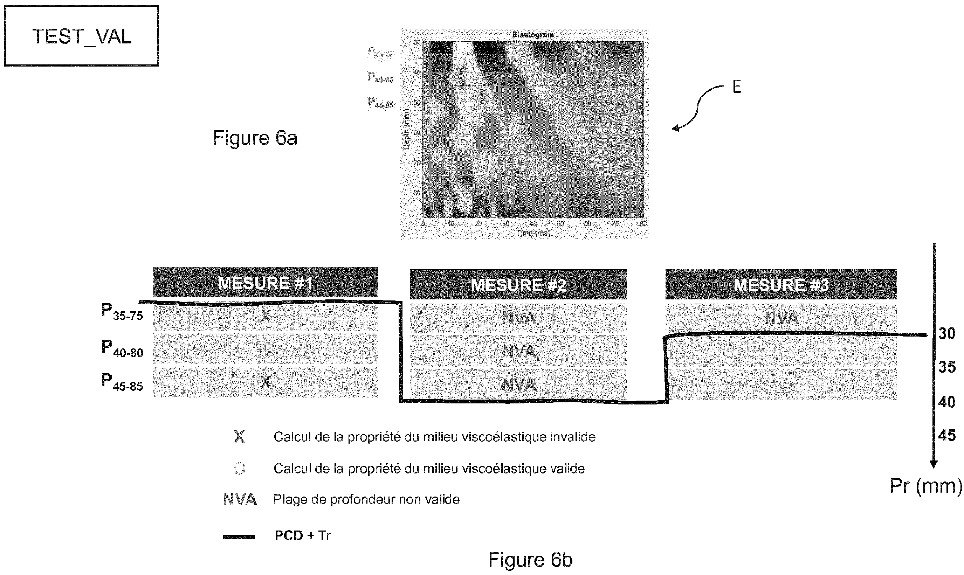

La figure 6a illustre l'étape TEST_VAL de détermination de la validité du calcul de la valeur de la propriété du milieu viscoélastique sur la ou les plages de profondeur de calcul valides. Pour chaque mesure M, le critère de qualité du calcul est calculé dans les plages de profondeurs valides.

Le critère de validité d'un calcul de la propriété est établi à partir d'un critère de qualité de l'élastogramme impulsionnel E.

La figure 6b illustre un exemple de validation des calculs des valeurs d'une propriété d'un milieu viscoélastique sous forme de tableau, construit à partir d'un élastogramme tel que celui représenté à la figure 6a. La construction d'un tableau tel que celui illustré à la figure 6b est un mode de réalisation de l'étape TEST_VAL de détermination de la validité de chaque calcul de la propriété en fonction des plages de profondeurs valides.

Le tableau de la figure 6b illustre le cas d'une séquence comprenant les mesures #1, #2, #3 pour trois plages de profondeur possibles : P[35-75], P[40-80] et P[45-85].

La ligne en trait continu représente la profondeur de la distance sonde-capsule PCD + Tr à la mesure #i.

Les lignes du tableau indiquent les résultats binaires de validité des calculs de la propriété pour chaque plage de profondeur P. Les traits inférieurs de chaque ligne correspondent à la profondeur palier à partir de laquelle la plage de profondeur devient invalide. Lorsque la plage de profondeur n'est pas valide, le critère de validité du calcul de la propriété n'est pas déterminé et est définie invalide.

Ce tableau illustre les conditions de validité du calcul de la valeur d'une propriété viscoélastique en fonction des variations de valeurs de la PCD.

Les critères de validité du calcul de la propriété de la figure 6b sont binaires et peuvent prendre deux valeurs « o » et « x ». La valeur « o » correspond à un calcul valide sur une plage de profondeur donnée, la valeur « x » correspond à un calcul non valide. L'invalidité d'une plage de profondeur est symbolisée par le sigle « NVA », symbolisant que la distance sonde capsule PCD ne respecte pas la condition suivante définie précédemment : Pi > PCD + Tr.

Les valeurs du critère de validité du calcul de la valeur de la propriété montrent que la mesure #1 est valide sur la plage de profondeur P[40-80] et non valide aux deux autres plages de profondeurs P[35-75] et P[45-85] indiquant une mauvaise qualité de l'élastogramme.

Les valeurs du critère de plage de profondeur indiquent que la mesure #2 est non valide sur toutes les plages de profondeurs. La position du trait plein noir montre la présence de la capsule du foie à l'intérieur des plages de profondeurs possibles lors de la réalisation de la mesure.

Les valeurs du critère de validité de plage de profondeur montrent que la mesure #3 est non valide sur la plage de profondeur P[35-75], à savoir la plage de profondeur la plus superficielle. La non validité du calcul de la propriété #3 en correspondance de la plage de profondeur P[35-75] est causée par la présence de la capsule du foie dans cette plage de profondeur.

Les étapes de détermination de validité de plage de profondeur TEST_PCD et de mesures TEST_VAL sont typiquement réalisées à l'aide de moyens de calcul tels qu'une mémoire et un microprocesseur présents dans le dispositif utilisé pour réaliser la mesure.

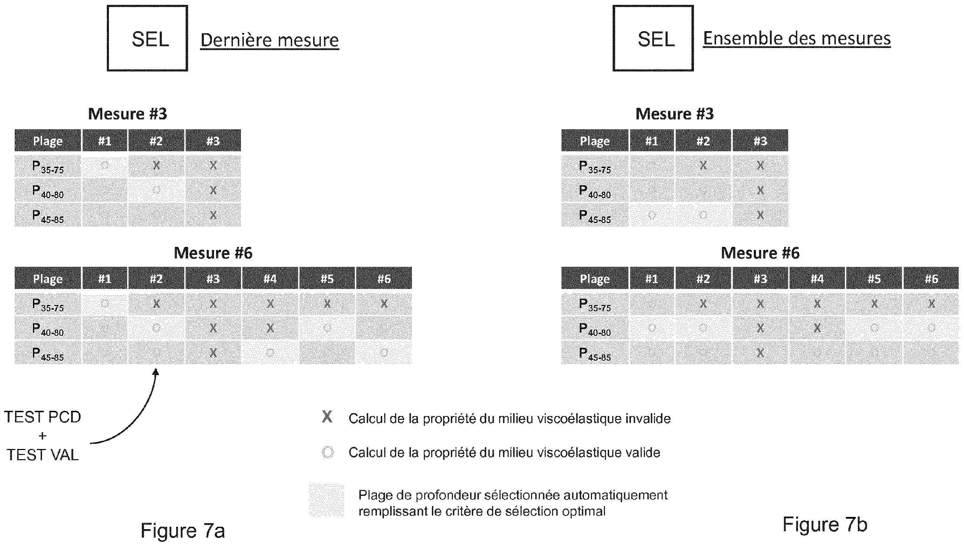

L'étape de sélection SEL automatique de la plage de profondeur remplissant le critère de sélection optimal lorsque la sélection de la plage optimale repose uniquement sur la mesure courante est illustrée à la figure 7a.

La figure 7a représente le cas d'une mesure répétée M = 6 fois sur P = 3 plages possibles. Les deux tableaux représentent l'historique des critères de validité des mesures aux moments des mesures #3 et #6 dans les trois plages de profondeur.

Chaque colonne représente les valeurs du critère de validité du calcul de la valeur de la propriété du milieu viscoélastique calculé pour une mesure #i donnée, chaque ligne correspondant à une des trois plages de profondeurs possibles.

Dans une plage de profondeur donnée, un calcul de la propriété peut être invalidé soit en raison d'une plage de profondeur invalide, soit par un critère de qualité de l'élastogramme inférieur à un seuil prédéterminé.

Pour chaque mesure, la plage de profondeur est automatiquement sélectionnée indépendamment des mesures précédentes, et apparaît sur fond clair dans les tableaux. D'une mesure à l'autre, l'historique de sélection de la plage de profondeur optimale ne change pas.

La valeur moyenne ou médiane de la propriété du milieu viscoélastique est calculée à partir de toutes les valeurs de la propriété viscoélastique valides, sur des plages de profondeur potentiellement différentes.

L'étape de sélection SEL automatique de la plage de profondeur remplissant le critère de sélection optimal lorsque la sélection de la plage optimale repose sur l'ensemble ou historique des mesures est illustrée à la figure 7b.

La figure 7b reprend l'exemple de la figure 7a, représentant le cas d'une mesure répétée M = 6 fois sur P = 3 plages possibles. Les deux tableaux représentent l'historique des critères de validité des mesures aux moments des mesures #3 et #6 dans les trois plages de profondeur.

Chaque colonne représente les valeurs du critère de validité d'un calcul de la valeur de la propriété calculée pour une mesure #i donnée, chaque ligne correspondant à une des trois plages de profondeurs possibles.

Dans une plage de profondeur donnée, un calcul de la propriété peut être invalidé soit en raison d'une plage de profondeur invalide, soit par un critère de qualité de l'élastogramme inférieur à un seuil prédéterminé.

Pour chaque mesure, la plage de profondeur est automatiquement sélectionnée et apparaît sur fond clair dans les tableaux. D'une mesure à l'autre, l'historique de sélection de la plage de profondeur est actualisé. La plage de profondeur sélectionnée de toutes les mesures précédentes est remplacée par la plage de profondeur optimale de la dernière mesure.

Par exemple, au moment de la mesure #3, la plage la plus profonde P[45-85] est sélectionnée pour toutes les mesures #1 à #3. Au moment de la mesure #6, le critère de sélection optimal est rempli pour la plage P[40-80], ce qui actualise et remplace toutes les sélections de plages précédentes par cette même plage de profondeur P[40-80].

La valeur moyenne ou médiane de la propriété du milieu viscoélastique est calculée à partir de toutes les valeurs de la propriété valides sur la même plage de profondeur.

Selon un mode de réalisation, si deux ou plusieurs plages de profondeurs fournissent le même critère de sélection optimal lors de l'étape de sélection SEL, la plage de profondeur la plus superficielle est choisie.

Avantageusement, cela permet de choisir la plage de profondeur la plus proche du transducteur ultrasonore et donc celle qui présente le rapport signal/bruit le plus grand.

Alternativement, selon un autre mode de réalisation, la plage de profondeur la plus profonde est choisie.

Avantageusement, cela permet de retenir la plage de profondeur la plus éloignée de la capsule du foie.

L'étape de sélection SEL est typiquement réalisée à l'aide de moyens de calcul tels qu'une mémoire et un microprocesseur présents dans le dispositif utilisé pour réaliser la mesure.

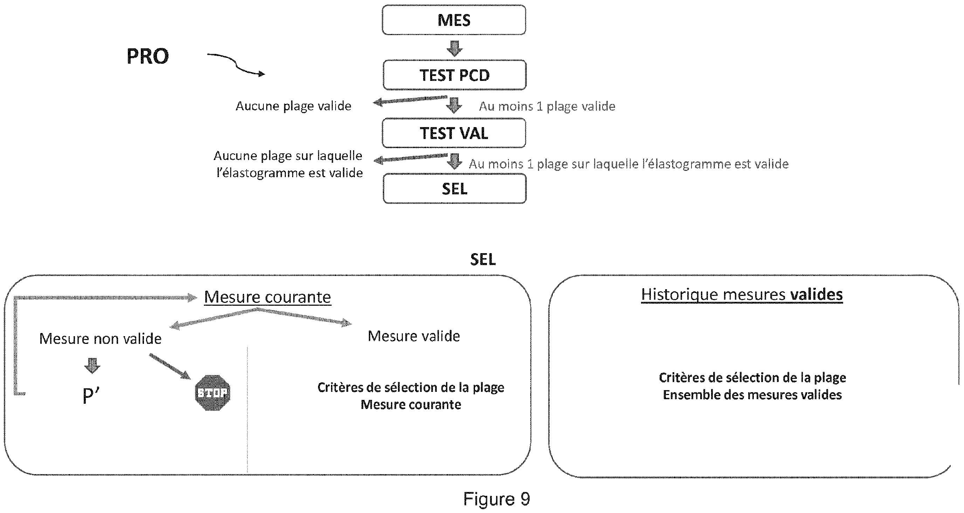

La figure 9 résume graphiquement les étapes de validation de plage de profondeur TEST_PCD, de validation du calcul de la valeur de la propriété du milieu viscoélastique TEST_VAL et de sélection SEL de la plage de profondeur optimale.

Selon un mode de réalisation, la sélection SEL de la plage de profondeur optimale repose uniquement sur la mesure courante.

Selon un autre mode de réalisation, la sélection SEL de la plage de profondeur optimale repose sur l'historique des mesures réalisées.

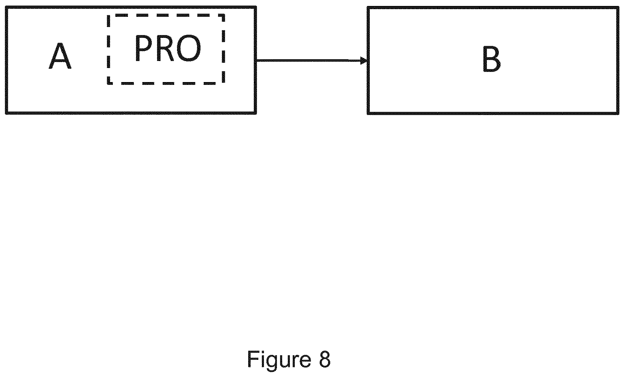

La figure 8 illustre schématiquement les étapes du procédé de mesure globale d'une propriété d'un milieu viscoélastique avec sélection automatique de la plage de profondeur de calcul, la sélection automatique de la plage de profondeur étant réalisée à l'aide du procédé PRO selon l'invention.

Le procédé de mesure comprend une première étape A de sélection automatique de la plage de profondeur de calcul en utilisant le procédé PRO selon l'invention. Lors de l'étape A, les valeurs de la propriété viscoélastique mesurées sont stockées dans une mémoire.

Le procédé de mesure globale comprend en outre une deuxième étape B de calcul global de la propriété viscoélastique à partir des valeurs calculées lors de l'étape A. Le calcul global peut être réalisé à l'aide d'une fonction de type moyenne ou médiane.

Selon un mode de réalisation, lors de l'étape B, la propriété du milieu viscoélastique est calculée à partir des valeurs de la propriété dont le calcul est valide et ont été réalisés lors de l'étape A.

Si le procédé PRO comporte la réalisation de M mesures, le calcul global de la propriété du milieu viscoélastique est réalisé à partir des valeurs de la propriété dont le calcul est valide et réalisé dans la ou les plages de profondeurs précédemment sélectionnées. Par exemple, la valeur de la propriété du milieu viscoélastique est la moyenne ou la médiane des valeurs de la propriété dont le calcul est valide obtenues sur la ou les plages de profondeur sélectionnées.

En pratique, dans le cas d'une sélection de plage de profondeur reposant sur la mesure courante uniquement, une fois que la plage de profondeur de la dernière mesure est sélectionnée, les M mesures correspondant à des plages de profondeurs indépendantes sont utilisées pour déterminer la valeur globale de la propriété mesurée. Par exemple, la valeur globale mesurée peut être une moyenne des valeurs calculées en correspondance de la plage sélectionnée.

Dans le cas d'une sélection de plage de profondeur reposant sur l'historique des mesures réalisées, une fois que la plage de profondeur de la dernière mesure est sélectionnée, les valeurs de la propriété des M mesures correspondant à la dernière plage retenue sont utilisées pour déterminer la valeur globale de la propriété. Par exemple, la valeur globale peut être une moyenne des valeurs calculées en correspondance de la plage choisie. Avantageusement, le procédé de mesure globale selon l'invention permet d'améliorer la reproductibilité et la fiabilité de la mesure d'une propriété viscoélastique en sélectionnant les valeurs de la propriété dont le calcul est valide sur la ou les plages de profondeur optimales pour la mesure.

Les mesures nécessaires à la mise en œuvre des procédés de sélection automatique d'une plage de profondeur et de mesure d'une propriété d'un milieu viscoélastique selon l'invention peuvent avantageusement être réalisées dans le cadre d'une mesure d'élastographie transitoire en utilisant un dispositif tel qu'un Fibroscan^{®}.

Dans ce cas la propriété du milieu viscoélastique est choisie dans un groupe de propriété comprenant : l'élasticité, le module d'Young, le module de cisaillement, la vitesse de propagation d'une onde de cisaillement à l'intérieur du milieu viscoélastique.

Les P plages possibles de profondeur de calcul sont fixées à l'intérieur de la plage de profondeur d'acquisition ultrasonore dans laquelle le milieu est observé. Cette zone d'observation dépend des propriétés de la sonde utilisée lors de l'examen. Comme illustré à la figure 1, il existe plusieurs choix de plages P de profondeurs de mesure.

Chaque mesure M mettant en œuvre le procédé PRO selon l'invention comporte le suivi de la propagation d'une onde de cisaillement transitoire à l'intérieur du milieu viscoélastique à caractériser. Pour ce faire, lors de la mesure #i, un élastogramme transitoire E est construit à partir du signal ultrasonore acquis durant la mesure d'élastographie impulsionnelle transitoire. L'élastogramme E permet à la fois de mesurer une des propriétés élastiques citées plus haut et de déterminer un critère de validité du calcul de la propriété dans une plage de profondeur définie.

Lors de la propagation de l'onde de cisaillement transitoire, des acquisitions ultrasonores sont générées avec un taux de répétition élevé pour suivre la propagation de l'onde de cisaillement transitoire. Les signaux réfléchis peuvent être également utilisés pour déterminer la distance sonde-capsule. La valeur de la distance sonde-capsule permet de calculer le critère de validité d'une plage de profondeur.

L'étape de sélection automatique de la plage de profondeur est réalisée à l'aide des moyens de calcul compris dans le dispositif utilisé pour réaliser la mesure d'élastographie transitoire. Les moyens de calculs comprennent par exemple une mémoire et un microprocesseur. La mémoire est configurée pour stocker les résultats des M mesures effectuées ainsi que les valeurs des différents critères de validité calculés par le microprocesseur.

Le dispositif utilisé pour réaliser la mesure d'élastographie transitoire comprend en outre des moyens d'affichage des résultats des procédés tels que la plage de profondeur de calcul choisie ou la valeur de la propriété du milieu viscoélastique.

Alternativement, la propriété du milieu peut être un paramètre d'atténuation ultrasonore tel qu'un paramètre d'atténuation contrôlée (CAP).

Un troisième objet de la présente invention est un dispositif de mesure d'une propriété d'un milieu viscoélastique avec sélection automatique de la plage de profondeur de calcul en utilisant le procédé selon l'invention, ledit dispositif comprenant :
- une sonde pour élastographie ;
- des moyens de calcul comprenant au moins une mémoire et un microprocesseur ;
ledit dispositif étant construit et agencé pour :
- Calculer, à partir du signal ultrasonore acquis à l'aide de la sonde pour élastographie et des moyens de calcul, la distance entre la sonde et la paroi délimitant le milieu viscoélastique (PCD) ;
- Calculer, à partir du signal ultrasonore acquis à l'aide de la sonde pour élastographie et des moyens de calcul, la propriété du milieu viscoélastique dans au moins une des P plages de profondeur de calcul possibles ;
- Déterminer la validité d'au moins une des P plages de profondeur de calcul, une plage de profondeur de calcul étant considérée valide si elle remplit un critère de validité calculé à partir de la distance entre la sonde et la paroi délimitant le milieu viscoélastique (PCD) ;
- Déterminer la validité du calcul de la valeur de la propriété sur la ou les plages de profondeur valides, une mesure étant considérée valide si elle remplit un critère de validité déterminé à partir de la qualité d'un élastogramme ;
- Sélectionner, parmi les plages de profondeurs valides comprenant au moins une mesure valide, une plage de profondeur remplissant un critère de sélection prédéterminé.
- Calculer, à partir des valeurs de la propriété dont le calcul est valide réalisées aux plages de profondeur sélectionnées, la valeur globale de la propriété viscoélastique, le calcul étant réalisé à l'aide d'une fonction mathématique de type médiane ou moyenne.

On entend par sonde pour élastographie une sonde comprenant au moins un transducteur ultrasonore. Un exemple de sonde pour élastographie est une sonde pour la mise en œuvre d'un procédé d'élastographie transitoire.

Le dispositif est configuré pour la mise en œuvre du procédé de sélection automatique de la plage de profondeur de calcul selon l'invention et pour calculer la valeur globale une propriété du milieu à l'intérieur des plages de profondeur sélectionnées.

Selon un mode de réalisation du dispositif selon l'invention, les moyens de calcul sont compris dans la sonde pour élastographie.

Selon un mode de réalisation, le dispositif selon l'invention comprend en outre des moyens d'affichage des résultats des mesures. Par exemple, les moyens d'affichage sont configurés pour afficher l'élastogramme mesuré, la plage de profondeur retenue pour la mesure et la propriété viscoélastique mesurée.

La figure 9 résume le procédé PRO de sélection automatique d'une plage de profondeur de calcul selon l'invention.

Lors de l'étape CALC, une sonde ultrasonore ou pour élastographie est utilisée pour calculer une propriété du milieu viscoélastique ainsi que la distance entre la sonde et la paroi du milieu viscoélastique PCD. La propriété du milieu viscoélastique et la PCD sont calculées à partir de tirs ultrasonores émis par la sonde et des ultrasons réfléchis par le milieu et détectés par la sonde.

Lors de l'étape TEST_PCD la validité des plages de profondeurs de calcul de la propriété viscoélastique est vérifiée. Une plage est considérée valide si elle est entièrement comprise à l'intérieur du milieu viscoélastique, à savoir si la distance sonde-paroi du milieu est inférieure aux bornes délimitant la plage de profondeur PCD < Pmin.

Selon un mode de réalisation, si aucune plage de profondeur n'est valide, le calcul de la valeur de la propriété du milieu viscoélastique est réalisé à la plage de profondeur suivante. Alternativement, le procédé PRO est arrêté.

Lors de l'étape TEST_VAL, la validité des calculs des valeurs de la propriété du milieu viscoélastique correspondant aux plages de profondeur valides est vérifiée. Un calcul est défini valide sur la base de la qualité de l'élastogramme associé à la mesure.

Lors de l'étape SEL, une plage de profondeur est sélectionnée parmi les plages de profondeur valides comprenant au moins un calcul de propriété valide. La sélection de la plage de profondeur est réalisée selon un critère prédéfini.

La figure 9 illustre deux modes de réalisation de l'étape de sélection SEL.

Selon un premier mode de réalisation, la sélection de la plage de profondeur de calcul repose uniquement sur la mesure courante ou dernière mesure réalisée. Dans ce cas, si plusieurs plages de profondeurs comprennent des valeurs de la propriété dont le calcul est valide, on applique un des critères de sélection suivants pour choisir une plage de profondeur :
- Meilleur rapport signal à bruit de l'élastogramme parmi toutes les plages de profondeurs ;
- Meilleure propagation de l'onde de cisaillement sur l'élastogramme (critère de qualité) parmi toutes les plages de profondeurs ;
- Meilleur critère d'homogénéité (LTT) parmi toutes les plages de profondeur.

Ce mode de réalisation est illustré aussi à la figure 7a.

Selon un deuxième mode de réalisation, la sélection de la plage de profondeur de calcul repose sur l'historique ou ensemble des mesures effectuées. Dans ce cas, la plage de profondeur sélectionnée est celle qui remplit un des critères suivants :
- Maximum du nombre de valeurs de la propriété dont le calcul est valide ;
- Minimum de dispersion de la valeur de la propriété moyenne ou médiane ;
- Meilleur rapport signal à bruit moyen ou médian de l'élastogramme parmi toutes les plages de profondeurs ;
- Meilleure propagation de l'onde de cisaillement sur l'élastogramme (critère de qualité moyen ou médian) parmi toutes les plages de profondeurs ;
- Meilleur critère d'homogénéité moyen ou médian (LTT) parmi toutes les plages de profondeurs.

Ce mode de réalisation est illustré aussi à la figure 7b.

## Revendications

1. Procédé (PRO) de sélection automatique d'une plage de profondeur de calcul d'une propriété d'un milieu viscoélastique, la plage de profondeur étant choisie parmi P plages possibles, P étant un nombre entier supérieur ou égal à 2, ledit procédé comprenant les étapes suivantes :
- Calculer (CALC), à partir d'un signal ultrasonore acquis à l'aide d'une sonde pour élastographie, la propriété du milieu viscoélastique dans au moins une des P plages de profondeur possibles et la distance entre la sonde et la paroi délimitant le milieu viscoélastique (PCD) ;
- Déterminer (TEST_PCD) la validité d'au moins une des P plages de profondeur de calcul, une plage de profondeur de calcul étant considérée valide si elle remplit un critère de validité calculé à partir de la distance entre la sonde et la paroi délimitant le milieu viscoélastique (PCD) ;
- Déterminer (TEST_VAL) la validité du calcul de la valeur de la propriété du milieu viscoélastique sur la ou les plages de profondeur de calcul valides, ledit calcul étant considéré valide s'il remplit un critère de validité calculé à partir de la qualité d'un élastogramme ;
- Sélectionner (SEL), parmi les plages de profondeur valides comprenant au moins un calcul valide de la propriété du milieu viscoélastique, une plage de profondeur remplissant un critère de sélection prédéterminé.

2. Procédé (PRO) de sélection automatique d'une plage de profondeur de calcul d'une propriété d'un milieu viscoélastique selon la revendication précédente **caractérisé en ce que** :
- Chaque plage de profondeur est délimitée par une première profondeur et une deuxième profondeur ;
- La plage de profondeur est définie valide si la distance entre la sonde et la paroi délimitant le milieu viscoélastique (PCD) est inférieure à la première et à la deuxième profondeur.

3. Procédé (PRO) de sélection automatique d'une plage de profondeur de calcul d'une propriété d'un milieu viscoélastique selon l'une des revendications précédentes **caractérisé en ce que**, lors de l'étape de calcul (CALC), la propriété du milieu viscoélastique est calculée à partir de M mesures réalisées dans au moins une des P plages de profondeur possibles, M étant un nombre entier supérieur ou égal à 2.

4. Procédé (PRO) de sélection automatique d'une plage de profondeur de calcul d'une propriété d'un milieu viscoélastique selon la revendication précédente **caractérisé en ce que** le critère de sélection de la plage de profondeur de calcul repose uniquement sur la dernière mesure réalisée.

5. Procédé (PRO) de sélection automatique d'une plage de profondeur de calcul d'une propriété d'un milieu viscoélastique selon la revendication précédente **caractérisé en ce que** la plage de profondeur sélectionnée lors de l'étape de sélection (SEL) est celle dans laquelle l'élastogramme comporte le plus grand rapport signal à bruit.

6. Procédé (PRO) de sélection automatique d'une plage de profondeur de calcul d'une propriété d'un milieu viscoélastique selon la revendication 4 **caractérisé en ce que** la plage de profondeur sélectionnée lors de l'étape de sélection (SEL) est celle pour laquelle l'élastogramme est de meilleure qualité.

7. Procédé (PRO) de sélection automatique d'une plage de profondeur de calcul d'une propriété d'un milieu viscoélastique selon la revendication 4 **caractérisé en ce que** la plage de profondeur sélectionnée lors de l'étape de sélection (SEL) est celle qui remplit un critère déterminé à partir de l'homogénéité du milieu.

8. Procédé (PRO) de sélection automatique d'une plage de profondeur de calcul d'une propriété d'un milieu viscoélastique selon la revendication 3 **caractérisé en ce que** le critère de sélection repose sur l'ensemble des mesures réalisées.

9. Procédé (PRO) de sélection automatique d'une plage de profondeur de calcul d'une propriété d'un milieu viscoélastique selon la revendication précédente **caractérisé en ce que** la plage de profondeur sélectionnée lors de l'étape de sélection (SEL) est celle qui minimise la dispersion entre les valeurs de la propriété calculées.

10. Procédé (PRO) de sélection automatique d'une plage de profondeur de calcul d'une propriété d'un milieu viscoélastique selon la revendication 8 **caractérisé en ce que** la plage de profondeur sélectionnée lors de l'étape de sélection (SEL) est celle qui maximise le nombre de calculs valides de la propriété.

11. Procédé (PRO) de sélection automatique d'une plage de profondeur de calcul d'une propriété d'un milieu viscoélastique selon la revendication 8 **caractérisé en ce que** la plage de profondeur sélectionnée lors de l'étape de sélection (SEL) est celle dans laquelle les élastogrammes calculés comportent le plus grand rapport signal à bruit.

12. Procédé (PRO) de sélection automatique d'une plage de profondeur de calcul d'une propriété d'un milieu viscoélastique selon la revendication 8 **caractérisé en ce que** la plage de profondeur sélectionnée lors de l'étape de sélection est celle pour laquelle les élastogrammes sont de meilleure qualité.

13. Procédé (PRO) de sélection automatique d'une plage de profondeur de calcul d'une propriété d'un milieu viscoélastique selon la revendication 8 **caractérisé en ce que** la plage de profondeur sélectionnée est celle qui remplit un critère de qualité déterminé à partir de l'homogénéité du milieu.

14. Procédé (PRO) de sélection automatique d'une plage de profondeur de calcul lors de la mesure d'une propriété d'un milieu viscoélastique selon la revendication 1 ou la revendication 2 **caractérisé en ce que**, si la plage de profondeur dans laquelle le calcul de la valeur de la propriété a été réalisée n'est pas valide, le calcul est réalisé à une plage plus profonde.

15. Procédé (PRO) de sélection automatique d'une plage de profondeur de calcul d'une propriété d'un milieu viscoélastique selon l'une des revendications précédentes **caractérisé en ce que** si, lors de l'étape de sélection (SEL), au moins deux plages de profondeurs remplissent le critère de sélection, la plage la plus superficielle est sélectionnée.

16. Procédé (PRO) de sélection automatique d'une plage de profondeur de calcul d'une propriété d'un milieu viscoélastique selon l'une des revendications précédentes **caractérisé en ce que** si, lors de l'étape de sélection (SEL), au moins deux plages de profondeurs remplissent le critère de sélection, la plage la plus profonde est sélectionnée.

17. Procédé de mesure globale d'au moins une propriété d'un milieu viscoélastique comprenant les étapes suivantes :
• Sélection automatique (A) de la plage de profondeur de calcul en utilisant le procédé (PRO) selon l'une des revendications 1 à 16 ;
• Calcul global (B) de la propriété du milieu viscoélastique à partir des valeurs de la propriété dont le calcul est valide aux plages de profondeur sélectionnées, le calcul étant réalisé à l'aide d'une fonction mathématique de type médiane ou moyenne.

18. Procédé de mesure globale d'au moins une propriété d'un milieu viscoélastique selon la revendication précédente **caractérisé en ce que** la propriété du milieu viscoélastique est choisie dans un groupe comprenant : l'élasticité, le module d'Young, le module de cisaillement, la vitesse de cisaillement à l'intérieur du milieu viscoélastique, un paramètre d'atténuation ultrasonore ou une combinaison de ces propriétés.

19. Dispositif pour la mesure d'une propriété d'un milieu viscoélastique avec sélection automatique de la plage de profondeur de calcul en utilisant le procédé selon l'une de revendication 17 ou 18, ledit dispositif comprenant :
• une sonde pour élastographie ;
• des moyens de calcul comprenant au moins une mémoire et un microprocesseur ;
ledit dispositif étant construit et agencé pour :
• Calculer, à partir du signal ultrasonore acquis à l'aide de la sonde pour élastographie et des moyens de calcul, la distance entre la sonde et la paroi délimitant le milieu viscoélastique (PCD) ;
• Calculer, à partir du signal ultrasonore acquis à l'aide de la sonde pour élastographie et des moyens de calcul, la valeur de la propriété du milieu viscoélastique dans au moins une des P plages de profondeur de calcul possibles ;
• Déterminer la validité d'au moins une des P plages de profondeur de calcul, une plage de profondeur de calcul étant considérée valide si elle remplit un critère de validité calculé à partir de la distance entre la sonde et la paroi délimitant le milieu viscoélastique (PCD) ;
• Déterminer la validité du calcul de la propriété du milieu viscoélastique sur la ou les plages de profondeur valides, ce calcul étant considéré valide s'il remplit un critère de validité déterminé à partir de la qualité d'un élastogramme ;
• Sélectionner, parmi les plages de profondeurs valides comprenant au moins un calcul de la propriété valide, une plage de profondeur remplissant un critère de sélection prédéterminé ;
• Calculer, à partir des valeurs de la propriété dont le calcul est valide aux plages de profondeur sélectionnées, la valeur globale de la propriété viscoélastique, la valeur globale étant calculée à l'aide d'une fonction mathématique de type médiane ou moyenne.

## Patentansprüche

1. Automatisches Auswahlverfahren (PRO) eines Tiefenbereichs zur Berechnung einer Eigenschaft eines viskoelastischen Milieus, wobei der Tiefenbereich aus P möglichen Bereichen ausgewählt wird, wobei P eine ganze Zahl größer als oder gleich 2 ist, wobei das genannte Verfahren die folgenden Schritte umfasst:
- Berechnen (CALC) der Eigenschaft des viskoelastischen Milieus in wenigstens einem der P möglichen Tiefenbereiche und der Entfernung zwischen der Sonde und der Wand, die das viskoelastische Milieu (PCD) begrenzt, ausgehend von einem Ultraschallsignal, das mithilfe einer Sonde zur Elastographie erworben wurde;
- Bestimmen (TEST_PCD) der Gültigkeit wenigstens einer der P Tiefenbereiche zur Berechnung, wobei ein Tiefenbereich zur Berechnung als gültig gilt, wenn er ein Gültigkeitskriterium erfüllt, das ausgehend von der Entfernung zwischen der Sonde und der Wand berechnet wird, die das viskoelastische Milieu (PCD) begrenzt;
- Bestimmen (TEST_VAL) der Gültigkeit der Berechnung des Wertes der Eigenschaft des viskoelastischen Milieus auf dem gültigen oder den gültigen Tiefenbereich(en) zur Berechnung, wobei die genannte Berechnung als gültig gilt, wenn sie ein Gültigkeitskriterium erfüllt, das anhand von der Qualität eines Elastogramms berechnet wird;
- Auswählen (SEL) eines Tiefenbereiches, der ein vorbestimmtes Auswahlkriterium erfüllt, aus den gültigen Tiefenbereichen, die wenigstens eine gültige Berechnung der Eigenschaft des viskoelastischen Milieus umfassen.

2. Automatisches Auswahlverfahren (PRO) eines Tiefenbereichs zur Berechnung einer Eigenschaft eines viskoelastischen Milieus gemäß dem voranstehenden Anspruch, **dadurch gekennzeichnet, dass**:
- jeder Tiefenbereich durch eine erste Tiefe und eine zweite Tiefe begrenzt wird;
- der Tiefenbereich als gültig definiert wird, wenn die Entfernung zwischen der Sonde und der Wand, die das viskoelastische Milieu (PCD) begrenzt, geringer ist als die erste und die zweite Tiefe.

3. Automatisches Auswahlverfahren (PRO) eines Tiefenbereichs zur Berechnung einer Eigenschaft eines viskoelastischen Milieus gemäß einem der voranstehenden Ansprüche, **dadurch gekennzeichnet, dass** die Eigenschaft des viskoelastischen Milieus beim Berechnungsschritt (CALC) ausgehend von M Messungen berechnet wird, die in wenigstens einem der P möglichen Tiefenbereiche ausgeführt werden, wobei M eine ganze Zahl größer als oder gleich 2 ist.

4. Automatisches Auswahlverfahren (PRO) eines Tiefenbereichs zur Berechnung einer Eigenschaft eines viskoelastischen Milieus gemäß dem voranstehenden Anspruch, **dadurch gekennzeichnet, dass** das Auswahlkriterium des Tiefenbereichs zur Berechnung ausschließlich auf der letzten ausgeführten Messung beruht.

5. Automatisches Auswahlverfahren (PRO) eines Tiefenbereichs zur Berechnung einer Eigenschaft eines viskoelastischen Milieus gemäß dem voranstehenden Anspruch, **dadurch gekennzeichnet, dass** der beim Auswahlschritt (SEL) ausgewählte Tiefenbereich der ist, bei dem das Elastogramm das größte Verhältnis des Nutz- zum Rauschsignal umfasst.

6. Automatisches Auswahlverfahren (PRO) eines Tiefenbereichs zur Berechnung einer Eigenschaft eines viskoelastischen Milieus gemäß Anspruch 4, **dadurch gekennzeichnet, dass** der beim Auswahlschritt (SEL) ausgewählte Tiefenbereich der ist, für den das Elastogramm von bester Qualität ist.

7. Automatisches Auswahlverfahren (PRO) eines Tiefenbereichs zur Berechnung einer Eigenschaft eines viskoelastischen Milieus gemäß Anspruch 4, **dadurch gekennzeichnet, dass** der beim Auswahlschritt (SEL) ausgewählte Tiefenbereich der ist, der ein Kriterium erfüllt, das ausgehend von der Homogenität des Milieus bestimmt wird.

8. Automatisches Auswahlverfahren (PRO) eines Tiefenbereichs zur Berechnung einer Eigenschaft eines viskoelastischen Milieus gemäß Anspruch 3, **dadurch gekennzeichnet, dass** das Auswahlkriterium auf der Gruppe der ausgeführten Messungen beruht.

9. Automatisches Auswahlverfahren (PRO) eines Tiefenbereichs zur Berechnung einer Eigenschaft eines viskoelastischen Milieus gemäß dem voranstehenden Anspruch, **dadurch gekennzeichnet, dass** der beim Auswahlschritt (SEL) ausgewählte Tiefenbereich der ist, der die Streuung zwischen den Werten der berechneten Eigenschaften minimiert.

10. Automatisches Auswahlverfahren (PRO) eines Tiefenbereichs zur Berechnung einer Eigenschaft eines viskoelastischen Milieus gemäß Anspruch 8, **dadurch gekennzeichnet, dass** der beim Auswahlschritt (SEL) ausgewählte Tiefenbereich der ist, der die Anzahl von gültigen Berechnungen der Eigenschaft maximiert.

11. Automatisches Auswahlverfahren (PRO) eines Tiefenbereichs zur Berechnung einer Eigenschaft eines viskoelastischen Milieus gemäß Anspruch 8, **dadurch gekennzeichnet, dass** der beim Auswahlschritt (SEL) ausgewählte Tiefenbereich der ist, bei dem die berechneten Elastogramme das größte Verhältnis des Nutz- zum Rauschsignal umfassen.

12. Automatisches Auswahlverfahren (PRO) eines Tiefenbereichs zur Berechnung einer Eigenschaft eines viskoelastischen Milieus gemäß Anspruch 8, **dadurch gekennzeichnet, dass** der beim Auswahlschritt ausgewählte Tiefenbereich der ist, bei dem die Elastogramme von bester Qualität sind.

13. Automatisches Auswahlverfahren (PRO) eines Tiefenbereichs zur Berechnung einer Eigenschaft eines viskoelastischen Milieus gemäß Anspruch 8, **dadurch gekennzeichnet, dass** der ausgewählte Tiefenbereich der ist, der ein Qualitätskriterium erfüllt, das ausgehend von der Homogenität des Milieus bestimmt wird.

14. Automatisches Auswahlverfahren (PRO) eines Tiefenbereichs zur Berechnung bei der Messung einer Eigenschaft eines viskoelastischen Milieus gemäß Anspruch 1 oder Anspruch 2, **dadurch gekennzeichnet, dass**, wenn der Tiefenbereich, in dem die Berechnung des Wertes der Eigenschaft ausgeführt wurde, nicht gültig ist, die Berechnung in einem tieferen Bereich ausgeführt wird.

15. Automatisches Auswahlverfahren (PRO) eines Tiefenbereichs zur Berechnung einer Eigenschaft eines viskoelastischen Milieus gemäß einem der voranstehenden Ansprüche, **dadurch gekennzeichnet, dass**, wenn beim Auswahlschritt (SEL) wenigstens zwei Tiefenbereiche das Auswahlkriterium erfüllen, der oberflächlichste Bereich ausgewählt wird.

16. Automatisches Auswahlverfahren (PRO) eines Tiefenbereichs zur Berechnung einer Eigenschaft eines viskoelastischen Milieus gemäß einem der voranstehenden Ansprüche, **dadurch gekennzeichnet, dass**, wenn beim Auswahlschritt (SEL) wenigstens zwei Tiefenbereiche das Auswahlkriterium erfüllen, der tiefste Bereich ausgewählt wird.

17. Umfassendes Messverfahren wenigstens einer Eigenschaft eines viskoelastischen Milieus, umfassend die folgenden Schritte:
• Automatische Auswahl (A) des Tiefenbereichs zur Berechnung unter Verwenden des Verfahrens (PRO) gemäß einem der Ansprüche 1 bis 16;
• Umfassende Berechnung (B) der Eigenschaft des viskoelastischen Milieus ausgehend von den Werten der Eigenschaft, deren Berechnung gültig ist, in den ausgewählten Tiefenbereichen, wobei die Berechnung mithilfe einer mathematischen Funktion vom medianen oder durchschnittlichen Typ ist.

18. Umfassendes Messverfahren wenigstens einer Eigenschaft eines viskoelastischen Milieus gemäß dem voranstehenden Anspruch, **dadurch gekennzeichnet, dass** die Eigenschaft des viskoelastischen Milieus aus einer Gruppe ausgewählt wird, umfassend: die Elastizität, das Youngsche Modul, das Abschermodul, die Abschergeschwindigkeit im Innern des viskoelastischen Milieus, einen Ultraschall-Abschwächungsparameter oder eine Kombination dieser Eigenschaften.

19. Vorrichtung für die Messung einer Eigenschaft eines viskoelastischen Moduls mit automatischer Auswahl des Tiefenbereichs zur Berechnung unter Verwenden des Verfahrens gemäß einem der Ansprüche 17 oder 18, wobei das genannte Verfahren umfasst:
• eine Sonde für die Elastographie
• Berechnungsmittel, umfassend wenigstens einen Speicher und einen Mikroprozessor;
wobei die genannte Vorrichtung aufgebaut und angeordnet ist zum:
• Berechnen der Entfernung zwischen der Sonde und der Wand, die das viskoelastische Milieu (PCD) begrenzt, ausgehend von dem Ultraschallsignal, das mithilfe der Sonde zur Elastographie und Berechnungsmitteln erworben ist;
• Berechnen des Wertes der Eigenschaft des viskoelastischen Milieus in wenigstens einem der P möglichen Tiefenbereiche zur Berechnung, ausgehend von dem Ultraschallsignal, das mithilfe der Sonde zur Elastographie und Berechnungsmitteln erworben ist;
• Bestimmen der Gültigkeit wenigstens eines der P Tiefenbereiche zur Berechnung, wobei ein Tiefenbereich zur Berechnung als gültig gilt, wenn er ein Gültigkeitskriterium erfüllt, das ausgehend von der Entfernung zwischen der Sonde und der Wand berechnet ist, die das viskoelastische Milieu (PCD) begrenzt;
• Bestimmen der Gültigkeit der Berechnung der Eigenschaft des viskoelastischen Milieus auf dem oder den gültigen Tiefenbereich(en), wobei diese Berechnung als gültig gilt, wenn sie ein Gültigkeitskriterium erfüllt, das ausgehend von der Qualität eines Elastogramms bestimmt ist;
• Auswählen eines Tiefenbereichs, der ein vorbestimmtes Auswahlkriterium erfüllt, aus den gültigen Tiefenbereichen, umfassend wenigstens eine Berechnung der gültigen Eigenschaft;
• Berechnen des umfassenden Wertes der viskoelastischen Eigenschaft, ausgehend von den Werten der Eigenschaft, deren Berechnung gültig ist, in den ausgewählten Tiefenbereichen, wobei der umfassende Wert mithilfe einer mathematischen Funktion vom medianen oder durchschnittlichen Typ berechnet ist.

## Claims

1. A method (PRO) for automatically selecting a depth range for calculating a property of a viscoelastic medium, the depth range being chosen from P possible ranges, P being an integer number greater than or equal to 2, said method comprising the following steps of:
- Calculating (CALC), from an ultrasound signal acquired using a probe for elastography, the property of the viscoelastic medium in at least one of the P possible depth ranges and the distance between the probe and the wall delimiting the viscoelastic medium (PCD);
- Determining (TEST_PCD) the validity of at least one of the P calculation depth ranges, a calculation depth range being considered as valid if it fulfills a validity criterion calculated from the distance between the probe and the wall delimiting the viscoelastic medium (PCD);
- Determining (TEST_VAL) the validity of the calculation of the value of the property of the viscoelastic medium over the valid calculation depth range or ranges, said calculation being considered as valid if it fulfills a validity criterion calculated from the quality of an elastogram;
- Selecting (SEL), from among the valid depth ranges comprising at least one valid calculation of the property of the viscoelastic medium, a depth range fulfilling a predetermined selection criterion.

2. The method (PRO) for automatically selecting a depth range for calculating a property of a viscoelastic medium according to the preceding claim, **characterised in that**:
- Each depth range is delimited by a first depth and a second depth;
- The depth range is defined as valid if the distance between the probe and the wall delimiting the viscoelastic medium (PCD) is less than the first and the second depths.

3. The method (PRO) for automatically selecting a depth range for calculating a property of a viscoelastic medium according to one of the preceding claims, **characterised in that**, during the calculation step (CALC), the property of the viscoelastic medium is calculated from M measurements made in at least one of the P possible depth ranges, M being an integer number greater than or equal to 2.

4. The method (PRO) for automatically selecting a calculation depth range for a property of a viscoelastic medium according to the preceding claim, **characterised in that** the selection criterion of the calculation depth range is based only on the last measurement made.

5. The method (PRO) for automatically selecting a calculation depth range for a property of a viscoelastic medium according to the preceding claim, **characterised in that** the depth range selected during the selection step (SEL) is that in which the elastogram has the greatest signal-to-noise ratio.

6. The method (PRO) for automatically selecting a depth range for calculating a property of a viscoelastic medium according to claim 4, **characterised in that** the depth range selected during the selection step (SEL) is that for which the elastogram is of the best quality.

7. The method (PRO) for automatically selecting a depth range for calculating a property of a viscoelastic medium according to claim 4, **characterised in that** the depth range selected during the selection step (SEL) is that which fulfills a criterion determined from the homogeneity of the medium.

8. The method (PRO) for automatically selecting a depth range for calculating a property of a viscoelastic medium according to claim 3, **characterised in that** the selection criterion is based on the complete set of measurements made.

9. The method (PRO) for automatically selecting a depth range for calculating a property of a viscoelastic medium according to the preceding claim, **characterised in that** the depth range selected during the selection step (SEL) is that which minimises dispersion between the calculated values of the property.

10. The method (PRO) for automatically selecting a depth range for calculating a property of a viscoelastic medium according to claim 8, **characterised in that** the depth range selected in the selection step (SEL) is that which maximises the number of valid calculations of the property.

11. The method (PRO) for automatically selecting a depth range for calculating a property of a viscoelastic medium according to claim 8, **characterised in that** the depth range selected during the selection step (SEL) is that in which the elastograms calculated have the greatest signal-to-noise ratio.

12. The method (PRO) for automatically selecting a depth range for calculating a property of a viscoelastic medium according to claim 8, **characterised in that** the depth range selected during the selection step is that for which the elastograms are of the best quality.

13. The method (PRO) for automatically selecting a depth range for calculating a property of a viscoelastic medium according to claim 8, **characterised in that** the depth range selected is that which fulfills a quality criterion determined from the homogeneity of the medium.

14. The method (PRO) for automatically selecting a calculation depth range upon measuring a property of a viscoelastic medium according to claim 1 or claim 2 **characterised in that**, if the depth range in which the calculation of the value of the property has been performed is not valid, the calculation is performed at a deeper range.

15. The method (PRO) for automatically selecting a depth range for calculating a property of a viscoelastic medium according to one of the preceding claims, **characterised in that** if, during the selection step (SEL), at least two depth ranges fulfill the selection criterion, the shallower range is selected.

16. The method (PRO) for automatically selecting a depth range for calculating a property of a viscoelastic medium according to one of the preceding claims, **characterised in that** if, during the selection step (SEL), at least two depth ranges fulfill the selection criterion, the deeper range is selected.

17. A method of global measurement of at least one property of a viscoelastic medium comprising the following steps of:
• Automatically selecting (A) the calculation depth range using the method (PRO) according to one of claims 1 to 16;
• Global calculation (B) of the property of the viscoelastic medium from the values of the property for which the calculation is valid at the selected depth ranges, the calculation being performed using a mathematical function of the median or mean type.

18. The method of global measurement of at least one property of a viscoelastic medium according to the preceding claim **characterised in that** the property of the viscoelastic medium is selected from a group comprising: elasticity, Young's modulus, shear modulus, shear rate within the viscoelastic medium, an ultrasound attenuation parameter or a combination of these properties.

19. A device for measuring a property of a viscoelastic medium with automatic selection of the calculation depth range using the method according to one of claims 17 or 18, said device comprising:
• a probe for elastography;
• calculation means comprising at least a memory and a microprocessor; said device being constructed and arranged to:
• Calculate, from the ultrasound signal acquired using the probe for elastography and the calculation means, the distance between the probe and the wall delimiting the viscoelastic medium (PCD);
• Calculate, from the ultrasound signal acquired using the probe for elastography and the calculation means, the value of the property of the viscoelastic medium in at least one of the P possible calculation depth ranges;
• Determine the validity of at least one of the P calculation depth ranges, a calculation depth range being considered as valid if it fulfills a validity criterion calculated from the distance between the probe and the wall delimiting the viscoelastic medium (PCD);
• Determine the validity of the calculation of the property of the viscoelastic medium over the valid depth range or ranges, this calculation being considered as valid if it fulfills a validity criterion determined from the quality of an elastogram;
• Select, among the valid depth ranges comprising at least one valid property calculation, a depth range fulfilling a predetermined selection criterion;
• Calculate, from the values of the property for which the calculation of which is valid at the selected depth ranges, the global value of the viscoelastic property, the global value being calculated using a mathematical function of the median or mean type.
